# EUROPEAN PATENT SPECIFICATION

(11) **EP 4 124 354 B1**
(45) Date of publication and mention of the grant of the patent: **22.04.2026**
(21) Application number: 21187681.8
(22) Date of filing: 26.07.2021
(51) Int. Cl.: A61M 5/20, A61M 5/24

(54) **DRUG ADMINISTRATION WITH SENSOR SYSTEM AND REMOTE COUPLING UNIT**
ARZNEIMITTELVERABREICHUNG MIT SENSORSYSTEM UND FERNGESTEUERTER KUPPLUNGSEINHEIT
ADMINISTRATION DE MÉDICAMENTS COMPRENANT UN SYSTÈME DE CAPTEUR ET UNE UNITÉ DE COUPLAGE À DISTANCE

(43) Date of publication of application: 01.02.2023
(73) Proprietor: Ypsomed AG, 3401 Burgdorf (CH)
(72) Inventor: Schüpbach, Simon, 3400 Burgdorf (CH); Baumgartner, Adrian, 2562 Port (CH)
(74) Representative: Meier Obertüfer, Jürg

(56) References cited:
- WO-A1-2018/064784
- WO-A1-2018/138192
- WO-A1-2019/087001
- CA-A1- 3 099 029
- KR-A- 20190 109 482
- US-A1- 2003 197 653
- US-A1- 2007 213 598
- US-A1- 2007 229 266
- US-A1- 2020 023 137

## Description

### BACKGROUND OF THE INVENTION

The present invention pertains to injection devices or medicament delivery devices for injecting, delivering, administering, infusing or dispensing flowable medical formulations, in particular comprising substances and/or liquids such as insulin or hormone preparations. It relates to an injection device, a monitoring unit attachable to the injection device, a drug administration system, a flexible circuit board, and a method of manufacturing an injection device in accordance with the independent patent claims.

### TECHNICAL FIELD OF THE INVENTION

A variety of diseases exist that require regular treatment by subcutaneous administration of a medicament, and a number of drug delivery devices, in particular administration and/or application devices, have been developed to support a patient in accurately and controllably delivering a well-defined amount of drug in a self-administration process. Delivery devices include injection devices that are removed from the injection site after each medication event or drug delivery process, in particular drug administration and/or application process, as well as infusion devices with a cannula or needle that remains in the skin of the patient for a prolonged period of time. Disposable delivery devices are adapted to deliver, in particular administer or apply, a drug from a container such as a pre-filled syringe that is not intended to be replaced or refilled by the patient. Reusable, semi-reusable, or hybrid delivery devices have at least a container and possibly also a container holder that may be replaced by the patient, or a cartridge that may be refilled, while some components of the device may be reused with the replaced or refilled drug container. By way of example, diabetes may be treated by administration of insulin by the patients themselves with the help of multi-variable-dose insulin injection pens or infusion pumps.

In the present context, the terms "substance", "drug", "medicament" and "medication" are to be understood to include any flowable medical formulation suitable for controlled delivery, administration and/or application through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin- containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

Fixed or variable dose disposable injection devices include single-dose injection devices such as auto injectors or patch injectors as well as multi-dose injection devices such as fixed or variable dose injectors. Auto-injectors automatically deliver a fixed or variable dose of liquid drug from a pre-filled syringe by means of a pre-tensioned injection spring biasing a piston rod and shifting a piston in a syringe barrel. Patch injectors or ready-to-use pre-filled wearable bolus injectors are patched or adhered to the skin of the patient in view of a single dose injection taking between thirty- seconds and several minutes. Fixed dose injectors have a single, non-variable dosage volume, or eventually provide a limited number of fixed, non-variable injection dosage volumes for the user to choose from. Variable dose injectors permit a user to change and/or influence an amount of drug to be administered.

Disposable injection devices may be complemented by a control or monitoring unit adapted to be successively attached to the device housings of plural disposable injection devices, thus forming a drug administration system. Said monitoring unit may constitute a or be part of a reusable electronic module or auxiliary device. The monitoring unit may, inter alia, serve to monitor the drug delivery process and/or a drug delivery status, in order to proactively prevent or retroactively recognize false handling of the device and to keep track of number and/or amount of doses already administered. In addition to generating data related to an instantaneous status, condition, or use of the injection device, information on the drug type, cartridge batch, and/or expiration date may be evaluated by the monitoring unit. To that end, the monitoring unit may comprise a delivery status sensing unit for tracking a progress of a medication event performed by means of the delivery device and/or for reading drug information that is stored on a machine-readable tag mounted to the device housing. The monitoring unit may further comprise a status indicator for signaling status and drug information to a user, and a wireless communication unit, in particular based on Bluetooth^{™} or WiFi, for communicating status and drug information to a nearby mobile device or medical gateway. All these units are supplied with electric power from an energy storing unit of the monitoring unit and/or the electronic module, wherein the monitoring unit and/or electronic module generally exclude any kind of electrically powered mechanical actuator or motor load. An exemplary electronic module with a sensing unit capable of discerning various operational states of a disposable auto-injector is disclosed in WO 2018/064784 A1, while a monitoring unit as referred to above is also described in more detail in WO 2019/186412 A1.

The electronic module of WO 2019/087001 A1 may also be regarded as a monitoring unit as described above, and is likewise configured to be attached to a disposable injection device. Said electronic module comprises a tag reader, in particular an RFID tag reader, for reading drug information, for example a batch number and/or an expiration date, from a tag or label, in particular an RFID tag, mounted to, or supported by, a device housing of the disposable injection device.

US 2007/213598 A1 relates to a system for communicating with a plurality of infusion pumps. The system includes a remote computer and a software to download or upload information from the infusion pumps. An antenna shema for an infusion pump with two diverse anennas to optimize wireless communication covarage is disclosed. According to the antenna shema the antennas are not only physically separated but a pattern diversity is applied by placing the two antennas orthogonally.

CA 3 099 029 A1 discloses a delivery pen and a pen cap. The pen cap includes a first NFC antenna and second NFC antenna placed adjacent to an analyte sensor. If the analyte sensor is located on a right arm of the user the first antenna is closer than the second antenna. If the analyte sensor is located on the left arm of the user the second antenna is closer. An NFC reader can be activated to alternate reading between the first antenna and the second antenna.

WO 2009/024562 A1 also describes an auxiliary device and a control circuit, both of which may be regarded as or as part of a monitoring unit as described above, said auxiliary device and/or control circuit being configured to be releasably attached to a first housing part coupled or being coupleable to a drug container, with the first housing part coupled to the drug container representing a disposable injection device as described above. A sensor system adapted to measure at least one physical or chemical parameter value related with the container and/or the fluid contained therein, may be attached to the container, comprised within the drug container or it may form an integral part of the container. The sensor system may comprise a sensor unit connected to or itself being an RFID (Radio Frequency IDentification) unit. The RFID enables the sensor unit to be supplied with energy without the need for wired connection as well as enables the sensor unit to transmit data such as a sensed value or a code wirelessly, which can then be received by the control circuit. More generally, the sensor system comprises a wireless transmitter, in particular an electrically operable transponder device, configured to emit data including the at least one measured parameter value in response to activation by a suitable field. The control circuitry, on the other hand, comprises activation means suitable for causing the electrically operable transponder device to emit the data including the at least one measured parameter value. The control circuitry further comprises receiving means for receiving the data thus emitted.

Activation means and transponder device, however, emit electric, magnetic and/or electromagnetic fields and/or waves which may interfere with and/or disturb (other) electronic circuitry, devices and systems located in a neighborhood of the activation means and/or the
transponder device. Such electronic circuitry, devices and systems may include the monitoring unit itself, in particular if it contains, e.g., a communication unit for wireless transmission of drug information and reception of evaluating information to and from an optional gateway device via Bluetooth^{™} Low Energy (BTLE) or equivalent short or near range wireless communication technology as described in WO 2019/087001 A1. It may, however, also affect other electronic circuitry, devices and systems independent from the injection device and the control circuit. This may pose serious problems, in particular in a hospital or similar medical care environment, where such other electronic circuitry, devices and systems may be comprised by diagnostic, monitoring, surveillance or treatment equipment, malfunction of which may endanger human health or even life.

Alternatively, a control or monitoring unit with the aforementioned sensor, indicator and communication functionalities may be an integral part of a reusable electronic delivery device and as such be integrated into a device housing of the delivery device comprising the reusable components. In this case, the electronic delivery device may be a reusable injection pen with a monitoring unit and a manually powered delivery drive requiring a user to manually provide the energy to move the piston or to charge a drive spring. The electronic delivery device may also be a reusable infusion pump with a monitoring unit and with a motor driving the piston automatically. All sensing, reading, evaluating, indicating, data processing, and communicating facilities of the monitoring unit are powered from an energy storing unit of the reusable delivery device.

### SUMMARY OF THE INVENTION

It is thus an objective of the invention to reduce a strength or intensity of electric or magnetic fields and/or electromagnetic radiation that drug delivery devices comprising wireless transmitters, in particular as part of RFID or NFC readers and/or tags, do emit.

The above objective and other objectives are solved by an injection device, a monitoring unit and a drug administration system in accordance with the independent claims 1 and 6, wherein further embodiments are defined in the dependent claims.

An injection device in accordance with the invention may comprise
- a drug reservoir;
- administration means for administering, in particular injecting, the drug through a skin of the subject in a drug administration process,
- a sensor system for determining, in particular measuring, a quantity, in particular a physical or chemical quantity, related to the drug administration process;
- a wireless transmitter configured to, upon activation, transmit information, in particular emit a signal, representative of the quantity, in particular to a receiver located at a distance from the transmitter;
- a remote coupling unit comprising
   ∘ a first antenna positioned relatively closer to the wireless transmitter,
   ∘ a second antenna positioned relatively farther from the wireless transmitter,
   ∘ electric connection means connecting the first antenna with the second antenna so that a signal received by the first antenna is re-emitted by the second antenna and vice versa.

The injection device may be of the kind described international patent application PCT/EP2021/052898, and in particular comprise some or all of the components described therein, and /or be assembled from some or all of these parts. The injection device may in particular be configured for an electronic module of the type as described in 2019/087001 A1 and/or an auxiliary device of the type as described in WO 2009/024562 A1 to be attached to it, in particular to its housing.

The injection device may have an at least essentially elongate shape, wherein a longitudinal direction exists and/or is defined by the elongate shape in which longitudinal direction dimensions of the elongate shape, in particular the injection device, are significantly larger than in any direction perpendicular to said longitudinal direction. The injection device may thus be said to extend in said longitudinal direction. The elongate shape, in particular the injection device, may be at least approximately rotationally symmetric, in particular at least approximately cylindrical, in which case the longitudinal direction may be parallel to a longitudinal axis of the elongate shape, in particular the injection device. The injection device may, in particular, be at least essentially pen-shaped.

The drug reservoir may comprise a syringe to which an injection needle may be attached, or may be formed integrally with the syringe.

The administration means may comprise one or more of a syringe holder, a plunger for displacing a drug contained in the reservoir through the injection needle, a spring for actuating the plunger, a cover sleeve for covering the needle prior to and/or after the injection to avoid injury, including unintentional skin penetration, to the subject or other persons, etc.

The sensor system may comprise a sensor for measuring, sensing or otherwise detecting a quantity related to a drug administration process, in particular a physical or chemical quantity related to the drug, in particular a physical or chemical property of the drug. By way of example, a temperature of the drug may be measured and/or monitored by means of a temperature sensor, in order to allow for verification that a drug about to be administered has been properly stored, i.e. without exceeding temperature limits for storage as usually defined and/or specified by a drug manufacturer. Measuring the temperature of the drug may also help to avoid discomfort or pain that may result when the drug is injected without having had enough time to warm up after being removed from refrigeration.

Alternatively and/or in addition, the sensor system may comprise a pressure sensor for measuring and/or monitoring a pressure of the drug in the reservoir, in particular during the drug administration process. Measuring the pressure as a function of time may allow to obtain information as to whether the drug was successfully and/or completely injected, or may help in determining an amount of drug that was injected.

Alternatively and/or in addition, the sensor system may comprise a chemical sensor, e.g. for measuring and/or monitoring a pH value of the drug in the reservoir.

Alternatively and/or in addition, the sensor system may comprise one or more sensors for determining quantities not directly related to the drug. By way of example, a skin contact sensor may be provided for determining whether a part of the injection device, in particular a distal end of a cover sleeve, is in contact with and/or positioned on skin of a subject to receive the drug. A status sensor which may be comprised by the sensor system may be configured to determine a property status of at least one element of the injection device, e.g. a position of the cover sleeve.

The quantity determined by the sensor system may be a continuous value representing a physical quantity, as e.g. in the case of a temperature, a pressure or a pH value as outlined above. The value may also be discrete, in particular binary, as may in particular be the case for a status of an element. By way of example, for the position of the cover sleeve, a first binary value, e.g. 0, may indicate that it is in an extended position (and covering the needle), whereas a second binary value, e.g. 1, may indicate that it is in a retracted position.

The wireless transmitter may be any means capable of interacting with a wireless receiver located at a distance from the wireless transmitter through electric or magnetic fields and/or electromagnetic radiation.

The wireless transmitter may be integrated with the sensor system, and vice versa.

The wireless transmitter may, in particular, be part of a transponder configured to, upon receiving a signal, emit a different signal in response, wherein the received signal may, in particular, provide an amount of energy, in particular electric energy, required for emitting the different signal. By way of example, the energy required may be obtained through induction and/or by energy harvesting, in particular from electromagnetic radiation incident onto the transponder.

Energy obtained by induction or energy harvesting may also be used to energize the sensor and/or the sensor system, i.e. to provide (electric) energy required for an operation of said sensor (system). Energizing the sensor in this manner obliviates the need for providing an independent energy storage like a battery, a pre-charge capacitor or similar that would otherwise be needed for the sensor (system) and/or the transmitter to work properly. The injection device may thus be realized free of any electric energy storage, in particular free of batteries and/or pre-charged capacitors for exclusively powering the transponder, the transmitter, the sensor or the sensor system.

The wireless transmitter and/or the transponder may be part of an NFC (near field communication) tag, or of an RFID (radio frequency identification) tag. Both RFID and NFC tags may be passive tags powered by energy from signals, in particular radio waves, used for interrogating the tags, from which energy may be obtained by induction and/or energy harvesting as described above, preferably by means of the wireless transmitter antenna operating in inductive mode. Energy obtained in this manner may also be used for powering the sensor and/or sensor system as described above.

If no energy storage is provided for energizing the wireless transmitter or the transponder comprising the latter, the sensor or the sensor system, one or more of the latter may be activated by a transceiver provided at a distance from the transponder and/or the wireless transmitter; in particular by a transceiver being comprised by an RFID reader or NFC reader, in particular as comprised by an electronic module of the type as described in 2019/087001 A1 and/or an auxiliary device of the type as described in WO 2009/024562 A1. The sensor, the sensor system, the wireless transmitter and/or the transponder may then remain inactive while and as long as the injection device is stored, e.g. as part of a stock maintained by a producer, a pharmacy, or a user of the drug and/or the injection device. Only when sufficient energy has been aggregated through induction or energy harvesting will the transponder be activated and the wireless transmitter be ready to emit a signal in response to the signal received by the transponder.

In embodiments, the injection device may also comprise an electric energy storage, in particular a battery and/or a pre-charged capacitor, for energizing the transponder, the transmitter, the sensor and/or the sensor system. The RFID or NFC tag together with the energy storage may thus constitute an active RFID or NFC tag, respectively. The energy storage may be integrated with the RFID or NFC tag. When the injection device comprises an electric energy storage for energizing the transponder, the transmitter, the sensor and/or the sensor system, activation by a transceiver as described above may not be necessary for the transponder, the transmitter, the sensor and/or the sensor system to function properly. A signal received from a transceiver provided at a distance from the transponder and/or the wireless transmitter; in particular by a transceiver being comprised by an RFID reader or NFC reader, in particular as comprised by an electronic module of the type as described in 2019/087001 A1 and/or an auxiliary device of the type as described in WO 2009/024562 A1, may nevertheless serve to trigger an operation of the transponder, the transmitter, the sensor and/or the sensor system. However, the transmitter, the sensor and/or the sensor system may be configured to be triggered by other events and/or circumstances that may occur during handling, manipulation and/or use of the injection device. By way of example, a switch may be provided as part of the injection device which switch is actuated when the injection device is subjected to certain manipulations, e.g. when a cap remover is separated from the injection device, or a cover sleeve moved from a first position to a second position. Alternatively, a switch may be provided as part of the injection device that may be actuated, in particular deliberately actuated, by a user of the injection device, e.g. by pressing a dedicated button, in particular when he is about to use the injection device to administer the drug contained therein.

The sensor, the sensor system and/or the wireless transmitter may be provided on a surface, in particular an inside surface, of a housing of the injection device. Any of them may be provided on an inner or outer surface of any one of various parts comprised by the injection device, or formed integrally with such part. Any of them may be provided on a surface of a syringe holder, or on an inner our outer surface of a syringe comprised by the injection device. Any, in particular all, of them may also be provided in a storage volume of the syringe, and optionally configured to float freely therein. Preferably, in such a configuration, the sensor, the sensor system and the wireless transmitter are encapsulated in a hermetic enclosure to prevent them from coming into direct contact with the drug.

The remote coupling unit may comprise a first antenna positioned in close proximity to the wireless transmitter, in particular to a transmitter antenna of the first wireless transmitter. The remote coupling unit and/or the first antenna may be provided on and/or as part of a different element of the injection device than wireless transmitter and/or the transponder. In particular, where the wireless transmitter and/or the transponder is provided on or as part of a drug container, in particular a syringe, the first antenna may be provided on a housing or on a drug container receptacle, in particular a syringe holder, of the injection device. If this is the case, a location of the first antenna may be chosen such that, when the injection device is assembled, the first antenna is located as closely to the wireless transmitter as the location on different elements of the injection device permits.

A position of the first antenna with respect to a longitudinal direction of the injection device may at least approximately be identical to a position with respect to the longitudinal direction of the wireless transmitter. Locations with respect to the longitudinal direction of the wireless transmitter and of the first antenna may at least partially overlap. More precisely, when the first antenna and/or the wireless transmitter are extended over first and second ranges of coordinates along the longitudinal direction, these ranges may at least partially overlap.

The second antenna may be located at a distance from the first antenna, in particular at a distance *d* larger, in particular significantly larger, than a distance between the first antenna and the wireless transmitter. The distance between the first antenna and the second antenna may primarily or at least almost exclusively extend in the longitudinal direction of the injection device. The distance *d* between the first antenna and the second antenna may be on the same order of magnitude as albeit smaller than, a length *l* of the injection device with respect to the longitudinal direction, in particular with *l >* 0.25d, preferably *l >* 0.5*d.*

The first antenna may comprise and/or be constituted by a first coil, comprising a first winding having one or several first turns, in particular when the wireless transmitter and/or the transponder are part of an NFC tag or an RFID tag. Similarly, the second antenna may comprise and/or be constituted by a second coil, comprising a second winding having one or several second turns. While such coils, frequently referred to as NFC coils or RFID coils, operate in the near field, in particular through near field magnetic induction, rather than by receiving and/or emitting far field electromagnetic waves, they are generally referred to as antennas, in particular when used in connection with NFC or RFID technology.

The first antenna may be connected with the second antenna by electric connection means, in particular an electrically conductive connection, in particular in series or in parallel. A matching network, in particular an impedance matching network, may be provided between the first antenna with the second antenna, and may be connected in series and/or in parallel with the first antenna and/or the second antenna to form an electric circuit together with the former. The matching network may, in particular be a passive network, in particular an LC, RC, RL or RLC network comprising capacitances, resistances and/or inductances connected in series and/or in parallel the first antenna and/or the second antenna. The matching network may be adapted to match a resonant frequency of the electric circuit at least approximately with a resonant frequency of the NFC tag or the RFID tag, and/or with an operating frequency of an NFC reader or RFID reader the NFC tag or the RFID tag is configured to interact with.

The second antenna my thus re-transmit information received by the first antenna, in particular re-emit a signal received by the first antenna; and vice versa. The re-emitted signal may be at least approximately identical with the received signal. However, the re-emitted signal may be one or more of phase shifted, time delayed, attenuated, amplified as compared to the received signal. Preferably, any information contained in the received signal should be maintained in the re-emitted signal.

A monitoring unit in accordance with the invention for monitoring a drug administration process executable by means of an injection device, in particular a disposable injection device, to which injection device the monitoring unit is adapted to be attached to or provided as a part of, said injection device comprising a wireless transmitter configured to transmit information, in particular emit a signal, related to the injection device, in particular to a drug administration process carried out by means of the injection device, may comprise:
- a wireless receiver configured to
   ∘ receive information related to the injection device, in particular to drug administration process carried out by means of said injection device, from said wireless transmitter;
- a remote coupling unit comprising:
   ∘ a first antenna positioned relatively farther from the wireless receiver,
   ∘ a second antenna positioned relatively closer to the wireless receiver,
   ∘ electric connection means connecting the first antenna with the second antenna so that a signal received by the first antenna is re-emitted by the second antenna and vice versa.

The monitoring unit may be of the kind described in WO 2019/186412 A1.

The monitoring unit may comprise an insertion bay configured to receive an injection device having an at least essentially elongate shape as described above. The insertion bay may have an at least essentially elongate hollow shape, wherein a longitudinal direction exists and/or is defined by the elongate hollow shape, in which longitudinal direction dimensions of the elongate hollow shape, in particular the insertion bay, are significantly larger than in any direction perpendicular to the longitudinal direction. The elongate hollow shape, in particular the insertion bay, may be at least approximately rotationally symmetric, in particular at least approximately cylindrical, in which case the longitudinal direction may be parallel to a longitudinal axis of the elongate hollow shape, in particular the insertion bay. When the injection device is inserted in the insertion bay, the longitudinal direction of the elongate hollow shape may extend parallel to, in particular coincide with, the longitudinal direction of the elongate shape of the injection device.

A retaining mechanism, in particular a latching mechanism, for temporarily retaining the injection device in the insertion bay may be provided as part of the injection device and/or the monitoring unit.

The wireless receiver may be any means capable of interacting with a wireless transmitter located at a distance from the wireless receiver through electric or magnetic fields and/or electromagnetic radiation.

The wireless receiver may, in particular, be part of a transceiver or of an NFC reader or of an RFID reader.

The remote coupling unit may comprise a second antenna positioned in close proximity to the wireless receiver, in particular to a receiver antenna of the first wireless receiver.

A drug administration system in accordance with the invention may comprise
- an injection device (1), in particular a disposable injection device, for administrating a drug to a subject in a drug administration process, comprising
   ∘ a drug reservoir;
   ∘ administration means for administering, in particular injecting, the drug through a skin of the subject,
   ∘ a sensor system for determining, in particular measuring, a quantity, in particular a physical or chemical quantity, related to the drug administration process;
   ∘ a wireless transmitter configured to, upon activation, transmit information, in particular emit a signal, representative of the quantity, in particular to a remote receiver;
- a monitoring unit (2) for monitoring a drug administration process executable by means of the injection device, said monitoring unit adapted to be attached to or provided as part of the injection device (1), said monitoring unit comprising:
   ∘ a wireless receiver configured to
      ▪ receive the information transmitted by the wireless transmitter, in particular the signal emitted by the wireless transmitter;
- a remote coupling unit comprising:
   ∘ a first antenna positioned closer to the wireless transmitter than to the wireless receiver,
   ∘ a second antenna positioned closer to the wireless receiver than to the wireless transmitter,
   ∘ electric connection means connecting the first antenna with the second antenna so that a signal received by the first antenna is re-emitted by the second antenna and vice versa.

The injection device, the monitoring unit and/or the remote coupling unit may be of the kind described above may have any of the optional characteristics and/or optional features described above in connection with the former elements.

The remote coupling unit may be provided integrally with and/or attached to the injection device, in particular to an outer surface of the injection device, in particular a housing of the injection device.

The remote coupling unit may comprise a second antenna positioned such that when the monitoring unit is attached to the injection device, in particular when the injection device is inserted into an insertion bay of the monitoring device, the wireless receiver, in particular a receiver antenna of the wireless receiver, is in close proximity with the second antenna. A location of the second antenna may, in particular, be chosen such that, when the monitoring unit is attached to the injection device, in particular when the injection device is inserted into an insertion bay of the monitoring device, the second antenna is located as closely to the wireless receiver as possible, in particular under the requirement that insertion of the injection device into the insertion be needs to be possible.

A position with respect to a longitudinal direction as defined by the injection device and/or as defined by the elongate hollow shape of the second antenna may at least approximately be identical to a position of the wireless receiver. Locations with respect to the longitudinal direction of the second antenna and the wireless receiver may at least partially overlap. More precisely, when the second antenna and/or the wireless receiver are extended over third and fourth ranges of coordinates along the longitudinal direction, said ranges may at least partially overlap.

The remote coupling unit may alternatively be provided integrally with and/or attached to the monitoring unit, in particular to a surface of the insertion bay.

Preferred embodiments and/or variations of the invention are presented in dependent claims.

### BRIEF DESCRIPTION OF THE DRAWINGS

Below, the subject matter of the invention will be explained with respect exemplary embodiments which are illustrated in the attached drawings, of which
Fig 1 depicts an exemplary drug administration system in accordance with the invention;
Fig 2 shows an exemplary injection device in accordance with the invention;
Fig. 3 illustrates an exemplary flexible circuit board 30 in accordance with the invention.

### DETAILED DESCRIPTION OF EXEMPLARY EMBODIMENTS

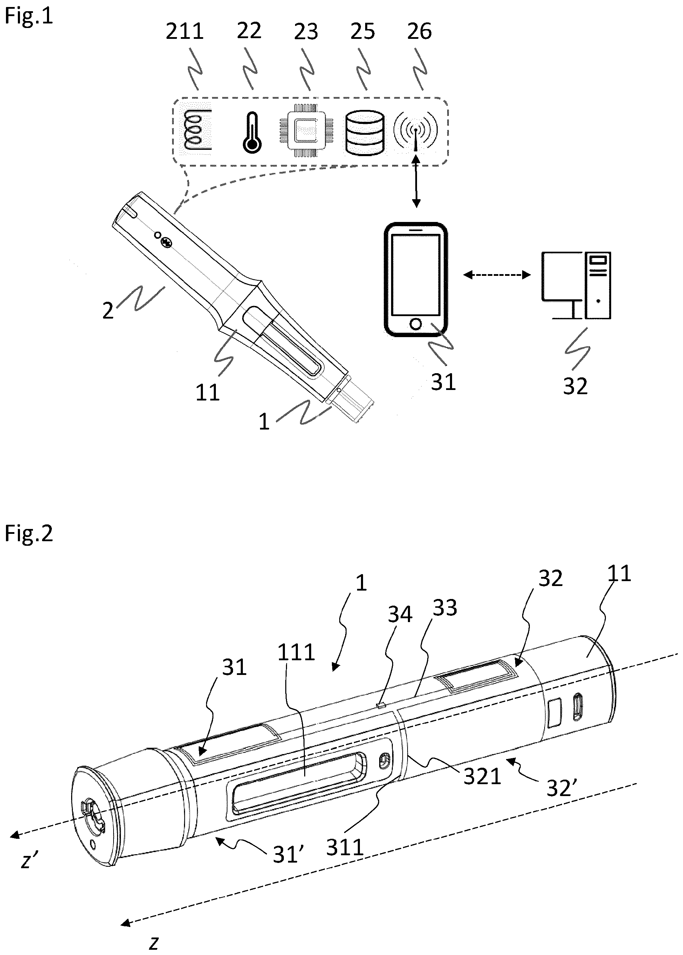

Fig.1 depicts an exemplary drug administration system comprising an exemplary monitoring unit 2 which is releasably attached to an injection device 1 and comprising a sensor system 22 for determining, in particular measuring, a quantity, in particular a physical quantity, in particular as described above. The monitoring unit comprises, inter alia, an RFID reader 211. The monitoring unit further comprises an integrated circuit 23 for controlling an operation of the RFID reader 211, the sensor system and/or other elements comprised by the monitoring unit. The monitoring unit further comprises a memory or data storage unit 25 adapted to store status or delivery information.

The monitoring unit 2 also comprises a communication unit 26 for wireless transmission of an injection status or drug status to the mobile device via Bluetooth Low Energy (BTLE) or equivalent short or near range wireless communication technology, which may be used for communication, in particular data exchange, with a mobile device 31 such as a smartphone or tablet device running a dedicated application program; or a laptop computer configured accordingly. The mobile device may in turn be communicatively connected via a data communication network, e.g. the Internet, to a remote server, cloud based computing facility, or expert system 32. The monitoring unit 2 has a rear, or proximal, part where some or all electronic components as described are located.

The integrated circuit 23, may, in particular, comprise a general purpose central processing unit (CPU), a graphics processing unit (GPU), a microcontroller, a reduced instruction set computer (RISC) processor, an application specific integrated circuit (ASIC), a programmable logic circuit (PLC), a field programmable gate array (FPGA), a digital signal processing (DSP) device, and/or any other circuit or processing device capable of providing and/or executing functionalities described above and/or below.

Fig. 2 shows an exemplary injection device 1 in accordance with the invention as hereinafter claimed. The injection device 1 has an elongate shape defining a longitudinal direction z. A first coil 31 is provided on an outside surface of a housing 11 of the injection device near a distal end of the injection device. A second coil 32 is provided on the outside surface of the housing 11 at a distance with respect to the longitudinal direction from the first coil 31 and near a proximal end of the injection device. Another first coil 31' (not visible in the figure) is located at a location corresponding to a position of the first coil 31 rotated by 180° about a longitudinal axis *z*' of the injection device parallel to the longitudinal direction z, and connected in series with the first coil 31 by a first electrically conducting connection 311. Likewise, another second coil 32' (not visible in the figure) is located at a location corresponding to a position of the second coil 32 rotated by 180° about the longitudinal axis *z*' of the injection device and connected in series with the second coil 32 by a second electrically conducting connection 321. The first coils 31, 31' and second coils 32, 32' form first and second antennas, respectively, which are electrically connected by electric connection means 33 comprising a matching network 34. A signal received by the first antenna is thus re-transmitted by the second antenna and vice versa. In particular, when any of the second coils 32 or 32' is subjected to, in particular traversed by, a time-dependent magnetic field, a corresponding, in particular essentially identical time-dependent magnetic field will be induced by the first coils 31 or 31', and vice versa.

Having first coils 31 and 31' located on opposite sides of the housing 11 as described above, allows for bringing the first antenna, in particular at least one coil of the first antenna, into close proximity with wireless transmitter comprised by the injection devise and located on an element of the injection device other than the housing, in particular independent of a relative orientation with respect to the longitudinal direction z and/or the longitudinal axis *z*' with which the elements are assembled.

Having second coils 32 and 32' likewise located on opposite sides of the housing 11 allows for bringing the second antenna, in particular at least one coil of the second antenna, into close proximity with wireless receiver comprised by the monitoring unit, independent of a relative orientation with respect to the longitudinal direction z and/or the longitudinal axis *z*' with which the monitoring unit 2 is attached to the injection device 1.

First and second coils 31, 31', 32, 32', first and second electrically conducting connection 311, 321, electric connection means 33 and matching network 34 may be provided on and/or as part of a flexible circuit board, sometimes also referred to as a flex circuit, in particular as a printed flexible circuit.

A window 111 provided in a wall of the housing allows to visually inspect a fill level of a drug container, in particular a syringe, of the injection device and located in the housing 11.

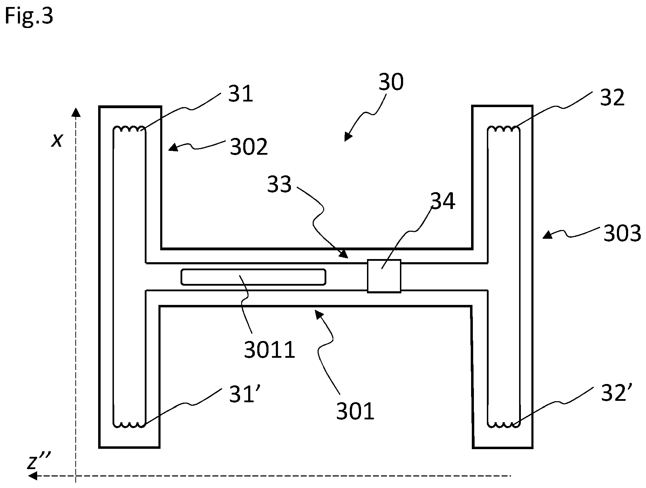

Fig. 3 illustrates an exemplary flexible circuit board 30 in accordance with the invention as hereinafter claimed. The flexible circuit board comprises two first coils 31 and 31' electrically connected in series to form a first antenna. The flexible circuit board further comprises two second coils 32 and 32' electrically connected in series to form a second antenna. First and second antenna are connected by electric connection means 33 comprising a matching network 34. The matching network 34 is provided on a first section 301 of the flexible circuit board having a first elongate shape defining a first longitudinal direction *z*". The first antenna is provided on a second section 302 of the flexible circuit board having a second elongate shape defining a second longitudinal direction x, which is perpendicular to the first longitudinal direction *z*". The second antenna is provided on a third section 303 of the flexible circuit board 30 having a second elongate shape defining a third longitudinal direction a third longitudinal direction at least essentially parallel to the second longitudinal direction x.

The two first coils 31 and 31' provided on the second section are spaced apart in or with respect to the second longitudinal direction x. Likewise, the two second coils 32 and 32' provided on the third section are spaced apart in or with respect to the second longitudinal direction x.

The flexible circuit board 30 is thus at least essentially H-shaped, with the second and third sections corresponding to the two parallel, vertical lines, and the first section.

The flexible circuit board 30 may be attached to an injection device, in particular to a disposable auto-injector as described in WO 2018064784 A1, in particular such that the first longitudinal direction *z*" extends parallel to a longitudinal direction and/or a longitudinal axis as defined by the injection device, in particular by the disposable auto-injector. A cut-out 3011 provided in the first section 301 of the flexible circuit board 30 may be placed over window 111.

The second and third sections 302, 303 may subsequently be wrapped around the injection device 1 in a circumferential direction.

This description and any accompanying drawings that illustrate aspects and embodiments of the present invention should not be taken as limiting the claims defining the protected invention. In other words, while the invention has been illustrated and described in detail in the drawings and foregoing description, such illustration and description are to be considered illustrative or exemplary and not restrictive. In some instances, well-known circuits, structures and techniques have not been shown in detail in order not to obscure the invention. Thus, it will be understood that changes and modifications may be made by those of ordinary skill within the scope and spirit of the following claims. In particular, the present invention covers further embodiments with any combination of features from different and/or individual embodiments as described above and below. Embodiments in accordance with the invention may, in particular, include further and/or additional features, elements, aspects, etc. not shown in the drawings or described above.

The disclosure also covers all further features shown in any Figure, individually, although they may not have been described in the afore or following description. Also, individual alternatives of the embodiments described in any Figure and the description and individual alternatives of features thereof can be disclaimed from the subject matter of the invention or from disclosed subject matter. The disclosure comprises subject matter consisting of the features defined in the claims or the exemplary embodiments as well as subject matter comprising said features.

The present disclosure also includes embodiments with any combination of features which are mentioned or shown above and/or below, in various embodiments or variants. It also includes individual features as shown in the Figures, even if they are shown there in connection with other features and/or are not mentioned above or below. The disclosure comprises embodiments which exclusively comprise the features described in the claims or the exemplary embodiments, as well as those which comprise additional other features. The steps of any method disclosed above or claimed below may preferably be carried out according the order in which they are presented, but may also be carried out in a different order.

Furthermore, in the claims the word "comprising" does not exclude other elements or steps, and the indefinite article "a" or "an" does not exclude a plurality. A single unit or step may fulfil the functions of several features recited in the claims. The mere fact that certain measures are recited in mutually different dependent claims does not indicate that a combination of these measures cannot be used to advantage. The terms "essentially", "substantially", "about", "approximately" and the like in connection with an attribute or a value particularly also define exactly the attribute or exactly the value, respectively. The term "about" in the context of a given numerate value or range may refer to a value or range that is, e.g., within 20%, within 10%, within 5%, or within 2% of the given value or range. Components described as coupled or connected may be electrically or mechanically directly coupled, or they may be indirectly coupled via one or more intermediate components. Any reference signs in the claims should not be construed as limiting the scope.

## Claims

1. An injection device (1), in particular a disposable injection device, for administrating a drug to a subject, said injection device comprising:
a. a drug reservoir;
b. administration means for administering, in particular injecting, the drug through a skin of the subject in a drug administration process;
c. a sensor system for determining a physical quantity related to the drug administration process;
d. a wireless transmitter configured to, upon activation, transmit information representative of the physical quantity to a first antenna (31);
e. a remote coupling unit (30) comprising
i. the first antenna (31) positioned relatively closer to the wireless transmitter to interact with the wireless transmitter,
ii. a second antenna (32) positioned relatively farther from the wireless transmitter,
iii. electric connection means (33) connecting the first antenna with the second antenna so that a signal received by the first antenna is re-emitted by the second antenna and vice versa.

2. The injection device of claim 1, further comprising:
a. an electric power source, in particular a battery or a pre-charged capacitor, for powering the wireless transmitter and/or the sensing unit;
b. switching means configured to be triggered before or when the drug is administered; said switching means further configured to connect the electric power source to the wireless transmitter and/or activate the wireless transmitter to transmit the information representative of the physical quantity.

3. The injection device of claim 1, wherein the wireless transmitter is comprised by a transponder configured to
a. be activated by a transceiver located at a distance from the wireless transmitter; and
b. upon activation, provide a response comprising the information representative of the physical quantity, in particular to the transceiver;
wherein, in particular, said transponder is part of a machine-readable tag, in particular an RFID tag or an NFC tag.

4. The injection device (1) of any of claims 1 to 3, **characterized in that** the injection device has an at least essentially elongate shape, in particular a pen-shape, said elongate shape defining a longitudinal direction, in particular a longitudinal axis, of the injection device (1).

5. The injection device (1) of claim 4, wherein a position of the first antenna with respect to the longitudinal direction of the injection device (1) overlaps with, in particular equals, a position of the transmitter with respect to the longitudinal direction of the injection device (1).

6. A monitoring unit (2) for monitoring a drug administration process executable by means of an injection device (1) according to claim 1, in particular a disposable injection device, to which the monitoring unit is adapted to be attached to, said injection device comprising a wireless transmitter configured to transmit information related to the injection device, in particular to a drug administration process carried out by means of the injection device, said monitoring unit comprising:
a. a wireless receiver configured to
i. receive information related to the injection device, in particular to drug administration process carried out by means of said injection device, from said wireless transmitter;
b. a remote coupling unit comprising:
i. a first antenna positioned relatively farther from the wireless receiver,
ii. a second antenna positioned relatively closer to the wireless receiver to interact with the wireless reciever,
iii. electric connection means connecting the first antenna with the second antenna so that a signal received by the first antenna is re-emitted by the second antenna and vice versa.

7. The monitoring unit (2) according to claim 6, wherein the wireless receiver is comprised by a transceiver configured to
a. activate a transponder located at a distance from the wireless receiver, in particular on or in the injection device, and configured to provide information related to the injection device,
b. receive a response containing information related to the injection device from said remote transponder
wherein, in particular, said transceiver is part of an RFID reader or an NFC reader.

8. The monitoring unit (2) according to claim 6 or 7, further comprising an insertion bay configured to receive an injection device (1) having an at least essentially elongate shape, in particular a pen-shape, said insertion bay having an at least essentially elongate hollow shape configured to receive the injection device, said elongate hollow shape defining a longitudinal direction, in particular a longitudinal axis, of the elongate hollow shape.

9. The monitoring unit (2) according to claim 8, wherein a position of the second antenna with respect to the longitudinal axis of the elongate hollow shape overlaps with, in particular equals, a position of the receiver with respect to the longitudinal direction of elongate hollow shape.

10. A drug administration system comprising
a. an injection device (1) according to any of claim 1 to 5;
b. a monitoring unit (2) for monitoring a drug administration process executable by means of the injection device (1), said monitoring unit adapted to be attached to the injection device, said monitoring unit comprising a wireless receiver configured to receive the information transmitted by the wireless transmitter and forwarded by the remote coupling unit.

11. The drug administration system of claim 10, wherein
a. the wireless transmitter is comprised by a transponder configured to
i. be activated by a transceiver located at a distance from the wireless transmitter, and
ii. upon activation, provide a response comprising information representative of the physical quantity, in particular to the transceiver;
wherein, in particular, said transponder is part of a machine-readable tag, in particular an RFID tag or an NFC tag, and/or
b. the wireless receiver is comprised by a transceiver configured to
i. activate a transponder located at a distance from the wireless receiver, in particular on or in the injection device, and configured to provide information related to the injection device,
ii. receive a response containing information related to the injection device from said transponder;
wherein, in particular, said transceiver is part of an RFID reader or an NFC reader.

12. The injection device (1) or the monitoring unit (2) according to one of the preceding claims, wherein
a. the first antenna comprises a first coil comprising one or several first conducting windings,
b. the second antenna comprises a second coil comprising one or several second conducting windings,
c. the first and second coil are electrically connected by means of a matching network (34), in particular an RL, RC, LC or RLC network.

13. The injection device (1) or the monitoring unit (2) according to one of the preceding claims, wherein
a. the first antenna comprises a plurality of first coils connected in parallel and/or in series, wherein
b. all coils of the plurality of first coils are located at least essentially at an identical position with respect to the longitudinal axis of the injection device (1) and/or the longitudinal axis of the elongate hollow shape,
c. all coils of the plurality of first coils are located at a different angle about the longitudinal axis of the injection device (1) and/or the longitudinal axis of the elongate hollow shape, in particular with one or more pairs of coils having coils located opposite from one another.

14. The injection device (1) or the monitoring unit (2) according to one of the preceding claims, wherein the first antenna, the second antenna and the matching network are provided on and/or by a flexible circuit board, in particular with resistors, capacitances and/or inductances provided integrally with the flexible circuit board, wherein the flexible circuit board preferably is free of discrete components.

15. The drug administration system of claim 10 or 11, wherein
a. an electric power source, in particular a battery or a pre-charged capacitor, for powering the wireless transmitter and/or the sensing unit;
b. switching means configured to be triggered before or when the drug is administered; said switching means further configured to connect the electric power source to the wireless transmitter and/or activate the wireless transmitter to transmit the information representative of the physical quantity.

## Patentansprüche

1. Injektionsvorrichtung (1), insbesondere eine Einweg-Injektionsvorrichtung, zum Verabreichen eines Arzneimittels an ein Subjekt, die Injektionsvorrichtung umfassend:
a. ein Arzneimittelreservoir;
b. Verabreichungsmittel zum Verabreichen, insbesondere Injizieren, des Arzneimittels durch eine Haut des Subjekts in einem Arzneimittelverabreichungsprozess;
c. ein Sensorsystem zum Bestimmen einer physikalischen Größe, bezogen auf den Arzneimittelverabreichungsprozess;
d. einen drahtlosen Sender, der konfiguriert ist, um, bei Aktivierung, Informationen, die für die physikalische Größe repräsentativ sind, an eine erste Antenne (31) zu senden;
e. eine entfernte Kopplungseinheit (30), umfassend
i. die erste Antenne (31), die relativ näher zu dem drahtlosen Sender positioniert ist, um mit dem drahtlosen Sender zu interagieren,
ii. eine zweite Antenne (32), die relativ weiter weg von dem drahtlosen Sender positioniert ist,
iii. elektrische Verbindungsmittel (33), die die erste Antenne mit der zweiten Antenne verbinden, sodass ein Signal, das durch die erste Antenne empfangen wird, durch die zweite Antenne wieder abgestrahlt wird und umgekehrt.

2. Injektionsvorrichtung nach Anspruch 1, ferner umfassend:
a. eine elektrische Leistungsquelle, insbesondere eine Batterie oder einen vorgeladenen Kondensator, zum Versorgen des drahtlosen Senders und/oder der Erfassungseinheit;
b. Schaltmittel, die konfiguriert sind, um ausgelöst zu werden, bevor oder wenn das Arzneimittel verabreicht wird; wobei die Schaltmittel ferner konfiguriert sind, um die elektrische Leistungsquelle mit dem drahtlosen Sender zu verbinden und/oder den drahtlosen Sender zu aktivieren, um die Informationen, die für die physikalische Größe repräsentativ sind, zu senden,

3. Injektionsvorrichtung nach Anspruch 1, wobei der drahtlose Sender in einem Transponder umfasst ist, der konfiguriert ist, um
a. durch einen Transceiver aktiviert zu werden, der sich in einem Abstand von dem drahtlosen Sender befindet; und
b. bei Aktivierung eine Antwort bereitzustellen, umfassend Informationen, die für die physikalische Größe repräsentativ sind, insbesondere an den Transceiver;
wobei insbesondere der Transponder Teil eines maschinenlesbaren Tags ist, insbesondere eines RFID-Tags oder eines NFC-Tags.

4. Injektionsvorrichtung (1) nach einem der Ansprüche 1 bis 3,
**dadurch gekennzeichnet, dass** die Injektionsvorrichtung eine mindestens im Wesentlichen längliche Form, insbesondere eine Stiftform, aufweist, wobei die längliche Form eine Längsrichtung, insbesondere eine Längsachse, der Injektionsvorrichtung (1) definiert.

5. Injektionsvorrichtung (1) nach Anspruch 4, wobei eine Position der ersten Antenne bezüglich der Längsrichtung der Injektionsvorrichtung (1) mit einer Position des Senders bezüglich der Längsrichtung der Injektionsvorrichtung (1) überlappt, insbesondere dieser entspricht.

6. Überwachungseinheit (2) zum Überwachen eines Arzneimittelverabreichungsprozesses, der mittels einer Injektionsvorrichtung (1) nach Anspruch 1, insbesondere einer Einweg-Injektionsvorrichtung ausführbar ist, an die die Überwachungseinheit angepasst ist, angebracht zu werden, die Injektionsvorrichtung umfassend einen drahtlosen Sender, der konfiguriert ist, um Informationen bezogen auf die Injektionsvorrichtung zu senden, insbesondere zu einem Arzneimittelverabreichungsprozess, der mittels der Injektionsvorrichtung vorgenommen wird, die Überwachungseinheit umfassend:
a. einen drahtlosen Empfänger, der konfiguriert ist zum
i. Empfangen von Informationen, bezogen auf die Injektionsvorrichtung, insbesondere auf den Arzneimittelverabreichungsprozess, der mittels der Injektionsvorrichtung von dem drahtlosen Sender vorgenommen wird;
b. eine entfernte Kopplungseinheit, umfassend:
i. eine erste Antenne, die relativ weiter weg von dem drahtlosen Empfänger positioniert ist,
ii. eine zweite Antenne, die relativ näher zu dem drahtlosen Empfänger positioniert ist, um mit dem drahtlosen Empfänger zu interagieren,
iii. elektrische Verbindungsmittel, die die erste Antenne mit der zweiten Antenne verbinden, sodass ein Signal, das durch die erste Antenne empfangen wird, durch die zweite Antenne wieder abgestrahlt wird und umgekehrt.

7. Überwachungseinheit (2) nach Anspruch 6, wobei der drahtlose Empfänger in einem Transceiver umfasst ist, der konfiguriert ist zum
a. Aktivieren eines Transponders, der sich in einem Abstand von dem drahtlosen Empfänger befindet, insbesondere an oder in der Injektionsvorrichtung, und konfiguriert ist, um Informationen bezogen auf die Injektionsvorrichtung bereitzustellen,
b. Empfangen einer Antwort, die Informationen bezogen auf die Injektionsvorrichtung von dem entfernten Transponder enthält,
wobei insbesondere der Transceiver Teil eines RFID-Lesers oder eines NFC-Lesers ist.

8. Überwachungseinheit (2) nach Anspruch 6 oder 7, ferner umfassend einen Einführschacht, der konfiguriert ist, um eine Injektionsvorrichtung (1), die mindestens eine im Wesentlichen längliche Form, insbesondere eine Stiftform aufweist, aufzunehmen, wobei der Einführschacht eine mindestens im Wesentlichen längliche hohle Form aufweist, die konfiguriert ist, um die Injektionsvorrichtung aufzunehmen, wobei die längliche hohle Form eine Längsrichtung, insbesondere eine Längsachse, der länglichen hohlen Form definiert.

9. Überwachungseinheit (2) nach Anspruch 8, wobei eine Position der zweiten Antenne bezüglich der Längsachse der länglichen hohlen Form mit einer Position des Empfängers bezüglich der Längsrichtung der länglichen hohlen Form überlappt, insbesondere dieser entspricht.

10. Arzneimittelverabreichungssystem, umfassend
a. eine Injektionsvorrichtung (1) nach einem der Ansprüche 1 bis 5;
b. eine Überwachungseinheit (2) zum Überwachen eines Arzneimittelverabreichungsprozesses, der mittels der Injektionsvorrichtung (1) ausführbar ist, wobei die Überwachungseinheit angepasst ist, an der Injektionsvorrichtung angebracht zu werden, die Überwachungseinheit umfassend einen drahtlosen Empfänger, der konfiguriert ist, um die Informationen, die durch den drahtlosen Sender gesendet und durch die entfernte Kopplungseinheit weitergeleitet werden, zu empfangen.

11. Arzneimittelverabreichungssystem nach Anspruch 10, wobei
a. der drahtlose in einem Transponder umfasst ist, der konfiguriert ist, um
i. durch einen Transceiver aktiviert zu werden, der sich in einem Abstand von dem drahtlosen Sender befindet, und
ii. bei Aktivierung eine Antwort bereitzustellen, umfassend Informationen, die für die physikalische Größe repräsentativ sind, insbesondere an den Transceiver;
wobei insbesondere der Transponder Teil eines maschinenlesbaren Tags ist, insbesondere eines RFID-Tags oder eines NFC-Tags, und/oder
b. der drahtlose Empfänger in einem Transceiver umfasst ist, der konfiguriert ist zum
i. Aktivieren eines Transponders, der sich in einem Abstand von dem drahtlosen Empfänger befindet, insbesondere an oder in der Injektionsvorrichtung, und konfiguriert ist, um Informationen bezogen auf die Injektionsvorrichtung bereitzustellen,
ii. Empfangen einer Antwort, die Informationen bezogen auf die Injektionsvorrichtung von dem entfernten Transponder enthält;
wobei insbesondere der Transceiver Teil eines RFID-Lesers oder eines NFC-Lesers ist.

12. Injektionsvorrichtung (1) oder die Überwachungseinheit (2) nach einem der vorstehenden Ansprüche, wobei
a. die erste Antenne eine erste Spule umfasst, umfassend eine oder mehrere erste leitende Windungen,
b. die zweite Antenne eine zweite Spule umfasst, umfassend eine oder mehrere zweite leitende Windungen,
c. die erste und zweite Spule mittels eines Anpassungsnetzwerks (34) elektrisch verbunden, insbesondere eines RL-, RC-, LC- oder RLC-Netzwerks.

13. Injektionsvorrichtung (1) oder die Überwachungseinheit (2) nach einem der vorstehenden Ansprüche, wobei
a. die erste Antenne eine Vielzahl von ersten Spulen umfasst, die parallel und/oder in Reihe geschaltet sind, wobei
b. sich alle Spulen der Vielzahl von ersten Spulen an wenigstens hauptsächlich einer identischen Position bezüglich der Längsachse der Injektionsvorrichtung (1) und/oder der Längsachse der länglichen hohlen Form befinden,
c. sich alle Spulen der Vielzahl von ersten Spulen in einem unterschiedlichen Winkel um die Längsachse der Injektionsvorrichtung (1) und/oder die Längsachse der länglichen hohlen Form befinden, insbesondere wobei ein oder mehrere Paare von Spulen Spulen aufweisen, die sich einander gegenüberliegend befinden.

14. Injektionsvorrichtung (1) oder die Überwachungseinheit (2) nach einem der vorstehenden Ansprüche, wobei die erste Antenne, die zweite Antenne und das Anpassungsnetzwerk auf und/oder durch eine flexible Leiterplatte bereitgestellt sind, insbesondere mit Widerständen, Kapazitäten und/oder Induktivitäten, die mit der flexiblen Leiterplatte integral bereitgestellt sind, wobei die flexible Leiterplatte vorzugsweise frei von diskreten Komponenten ist.

15. Arzneimittelverabreichungssystem nach Anspruch 10 oder 11, wobei
a. eine elektrische Leistungsquelle, insbesondere eine Batterie oder einen vorgeladenen Kondensator, zum Versorgen des drahtlosen Senders und/oder der Erfassungseinheit;
b. Schaltmittel, die konfiguriert sind, um ausgelöst zu werden, bevor oder wenn das Arzneimittel verabreicht wird; wobei die Schaltmittel ferner konfiguriert sind, um die elektrische Leistungsquelle mit dem drahtlosen Sender zu verbinden und/oder den drahtlosen Sender zu aktivieren, um die Informationen, die für die physikalische Größe repräsentativ sind, zu senden,

## Revendications

1. Dispositif d'injection (1), en particulier dispositif d'injection jetable, pour l'administration d'un médicament à un sujet, ledit dispositif d'injection comprenant :
a. un réservoir de médicament ;
b. un moyen d'administration permettant d'administrer, en particulier d'injecter, le médicament à travers une peau du sujet dans un processus d'administration de médicament;
c. un système de capteur permettant de déterminer une quantité physique liée au procédé d'administration de médicament ;
d. un émetteur sans fil configuré pour, une fois activé, transmettre des informations représentatives de la quantité physique à une première antenne (31) ;
e. une unité de couplage à distance (30) comprenant
i. la première antenne (31) positionnée relativement plus près de l'émetteur sans fil pour interagir avec l'émetteur sans fil,
ii. une seconde antenne (32) positionnée relativement plus loin de l'émetteur sans fil,
iii. un moyen de connexion électrique (33) connectant la première antenne à la seconde antenne de sorte qu'un signal reçu par la première antenne est réémis par la seconde antenne et vice versa.

2. Dispositif d'injection selon la revendication 1, comprenant en outre :
a. une source d'énergie électrique, en particulier une batterie ou un condensateur préchargé, pour alimenter l'émetteur sans fil et/ou l'unité de détection ;
b. un moyen de commutation configuré pour être déclenché avant ou pendant l'administration du médicament ; lesdits moyens de commutation étant en outre configurés pour connecter la source d'énergie électrique à l'émetteur sans fil et/ou activer l'émetteur sans fil pour transmettre les informations représentatives de la quantité physique.

3. Dispositif d'injection selon la revendication 1, dans lequel l'émetteur sans fil est constitué d'un transpondeur configuré pour
a. être activé par un émetteur-récepteur situé à une certaine distance de l'émetteur sans fil ; et
b. une fois activé, fournir une réponse comprenant les informations représentatives de la grandeur physique, en particulier à l'émetteur-récepteur ;
dans lequel, en particulier, ledit transpondeur fait partie d'une étiquette lisible par machine, en particulier une étiquette RFID ou une étiquette NFC.

4. Dispositif d'injection (1) selon l'une quelconque des revendications 1 à 3, **caractérisé en ce que** le dispositif d'injection a une forme au moins essentiellement allongée, en particulier une forme de stylo, ladite forme allongée définissant une direction longitudinale, en particulier un axe longitudinal, du dispositif d'injection (1).

5. Dispositif d'injection (1) selon la revendication 4, dans lequel une position de la première antenne par rapport à la direction longitudinale du dispositif d'injection (1) chevauche, en particulier est égale à, une position de l'émetteur par rapport à la direction longitudinale du dispositif d'injection (1).

6. Unité de surveillance (2) destinée à surveiller un procédé d'administration de médicament exécutable au moyen d'un dispositif d'injection (1) selon la revendication 1, en particulier un dispositif d'injection jetable, auquel l'unité de surveillance est destinée à être fixée, ledit dispositif d'injection comprenant un émetteur sans fil configuré pour transmettre des informations relatives au dispositif d'injection, en particulier à un procédé d'administration de médicament mis en œuvre au moyen du dispositif d'injection, ladite unité de surveillance comprenant :
a. un récepteur sans fil configuré pour
i. recevoir dudit émetteur sans fil des informations relatives au dispositif d'injection, en particulier au procédé d'administration de médicament mis en œuvre au moyen dudit dispositif d'injection ;
b. une unité de couplage à distance comprenant :
i. une première antenne positionnée relativement plus loin du récepteur sans fil,
ii. une seconde antenne positionnée relativement plus près du récepteur sans fil pour interagir avec le récepteur sans fil,
iii. un moyen de connexion électrique connectant la première antenne à la seconde antenne de sorte qu'un signal reçu par la première antenne est réémis par la seconde antenne et vice versa.

7. Unité de surveillance (2) selon la revendication 6, dans laquelle le récepteur sans fil est constitué d'un émetteur-récepteur configuré pour
a. activer un transpondeur situé à une certaine distance du récepteur sans fil, en particulier sur ou dans le dispositif d'injection, et configuré pour fournir des informations relatives au dispositif d'injection,
b. recevoir dudit transpondeur à distance une réponse contenant des informations relatives au dispositif d'injection
dans laquelle, en particulier, ledit émetteur-récepteur fait partie d'un lecteur RFID ou d'un lecteur NFC.

8. Unité de surveillance (2) selon la revendication 6 ou 7, comprenant en outre une baie d'insertion conçue pour recevoir un dispositif d'injection (1) ayant une forme au moins essentiellement allongée, en particulier une forme de stylo, ladite baie d'insertion ayant une forme creuse au moins essentiellement allongée conçue pour recevoir le dispositif d'injection, ladite forme creuse allongée définissant une direction longitudinale, en particulier un axe longitudinal, de la forme creuse allongée.

9. Unité de surveillance (2) selon la revendication 8, dans laquelle une position de la seconde antenne par rapport à l'axe longitudinal de la forme creuse allongée chevauche, en particulier est égale à, une position du récepteur par rapport à la direction longitudinale de la forme creuse allongée.

10. Système d'administration de médicament comprenant
a. un dispositif d'injection (1) selon l'une quelconque des revendications 1 à 5 ;
b. une unité de surveillance (2) pour la surveillance d'un procédé d'administration de médicament exécutable au moyen du dispositif d'injection (1), ladite unité de surveillance étant destinée à être fixée au dispositif d'injection, ladite unité de surveillance comprenant un récepteur sans fil configuré pour recevoir les informations transmises par l'émetteur sans fil et retransmises par l'unité de couplage à distance.

11. Système d'administration de médicament selon la revendication 10, dans lequel
a. l'émetteur sans fil est constitué d'un transpondeur configuré pour
i. être activé par un émetteur-récepteur situé à une certaine distance de l'émetteur sans fil, et
ii. une fois activé, fournir une réponse comprenant des informations représentatives de la grandeur physique, en particulier à l'émetteur-récepteur ;
dans lequel, en particulier, ledit transpondeur fait partie d'une étiquette lisible par machine, en particulier une étiquette RFID ou une étiquette NFC, et/ou
b. le récepteur sans fil est constitué d'un émetteur-récepteur configuré pour
i. activer un transpondeur situé à une certaine distance du récepteur sans fil, en particulier sur ou dans le dispositif d'injection, et configuré pour fournir des informations relatives au dispositif d'injection,
ii. recevoir dudit transpondeur une réponse contenant des informations relatives au dispositif d'injection ;
dans laquelle, en particulier, ledit émetteur-récepteur fait partie d'un lecteur RFID ou d'un lecteur NFC.

12. Dispositif d'injection (1) ou unité de surveillance (2) selon l'une des revendications précédentes, dans lequel
a. la première antenne comprend une première bobine comprenant un ou plusieurs premiers enroulements conducteurs,
b. la seconde antenne comprend une seconde bobine comprenant un ou plusieurs seconds enroulements conducteurs,
c. les première et seconde bobines sont connectées électriquement au moyen d'un réseau d'adaptation (34), en particulier un réseau RL, RC, LC ou RLC.

13. Dispositif d'injection (1) ou unité de surveillance (2) selon l'une des revendications précédentes, dans lequel
a. la première antenne comprend une pluralité de premières bobines connectées en parallèle et/ou en série, dans lequel
b. toutes les bobines de la pluralité de premières bobines sont situées au moins essentiellement au niveau d'une position identique par rapport à l'axe longitudinal du dispositif d'injection (1) et/ou à l'axe longitudinal de la forme creuse allongée,
c. toutes les bobines de la pluralité de premières bobines sont situées à un angle différent autour de l'axe longitudinal du dispositif d'injection (1) et/ou de l'axe longitudinal de la forme creuse allongée, en particulier avec une ou plusieurs paires de bobines ayant des bobines situées à l'opposé l'une de l'autre.

14. Dispositif d'injection (1) ou unité de surveillance (2) selon l'une des revendications précédentes, dans lequel la première antenne, la seconde antenne et le réseau d'adaptation sont fournis sur et/ou par une carte de circuit flexible, en particulier avec des résistances, capacités et/ou des inductances fournies intégralement avec la carte de circuit flexible, dans lequel la carte de circuit flexible est de préférence exempte de composants distincts.

15. Système d'administration de médicament selon la revendication 10 ou 11, dans lequel
a. une source d'énergie électrique, en particulier une batterie ou un condensateur préchargé, pour alimenter l'émetteur sans fil et/ou l'unité de détection ;
b. un moyen de commutation configuré pour être déclenché avant ou pendant l'administration du médicament ; lesdits moyens de commutation étant en outre configurés pour connecter la source d'énergie électrique à l'émetteur sans fil et/ou activer l'émetteur sans fil pour transmettre les informations représentatives de la quantité physique.
